# EUROPÄISCHE PATENTSCHRIFT

(11) **EP 2 918 597 B1**
(45) Veröffentlichungstag und Bekanntmachung des Hinweises auf die Patenterteilung: **19.07.2017**
(21) Anmeldenummer: 15158761.5
(22) Anmeldetag: 12.03.2015
(51) Int. Cl.: C07K 1/13, C07K 1/26, G01N 27/447

(54) **VERFAHREN UND KIT ZUM ÜBERWACHEN DER PROBENVORBEREITUNG, VON CHEMISCHEN MARKIERUNGSREAKTIONEN UND VON ANTIKÖRPERREAKTIONEN**
METHOD AND KIT FOR MONITORING SAMPLE PREPARATION OF CHEMICAL MARKING REACTIONS AND ANTIBODY RESPONSES
PROCÉDÉ ET KIT DE SURVEILLANCE DE PRÉPARATION D'ÉCHANTILLONS, DE RÉACTIONS DE MARQUAGE CHIMIQUE ET DE RÉACTIONS D'ANTICORPS

(30) Priorität: 12.03.2014 DE 102014204514
(43) Veröffentlichungstag der Anmeldung: 16.09.2015
(73) Patentinhaber: NH DyeAGNOSTICS GmbH, 06120 Halle (Saale) (DE)
(72) Erfinder: Heise, Jana, 06108 Halle (Saale) (DE)
(74) Vertreter: Maikowski & Ninnemann Patentanwälte Partnerschaft mbB

(56) Entgegenhaltungen:
- US-A1- 2009 272 649
- Kathryn S Lilley and David B Friedmann: "All about DIGE: quantification technology for differential-display 2D-gel proteomics", Expert Review of Proteomics, Bd. 1, Nr. 4 1. Januar 2004 (2004-01-01), Seiten 1-9, XP055201526, DOI: 10.1586/14789450.1.4.1 Gefunden im Internet: URL:http://citeseerx.ist.psu.edu/viewdoc/d ownload;jsessionid=6721D6DA618EAD33B0D2CD7 087E6F92B?doi=10.1.1.455.2565&rep=rep1&typ e=pdf [gefunden am 2015-07-10]
- "Fluorescence 2-D difference gel electrophoresis (2-D DIGE) using Ettan DIGE system and mass spectrometry proteomic analysis of Escherichia coli", Application Note, 1. Februar 2003 (2003-02-01), Seiten 1-2, XP055201273, Gefunden im Internet: URL:https://www.gelifesciences.com/gehcls_ images/GELS/Related Content/Files/1314742967685/litdoc18117081 AA_20110831003638.pdf [gefunden am 2015-07-09]
- NILESH S TANNU ET AL: "Two-dimensional fluorescence difference gel electrophoresis for comparative proteomics profiling", NATURE PROTOCOLS, Bd. 1, Nr. 4, 1. November 2006 (2006-11-01), Seiten 1732-1742, XP055201320, ISSN: 1754-2189, DOI: 10.1038/nprot.2006.256
- OLA KRISTOFFER ÖYE ET AL: "Gel2DE - A software tool for correlation analysis of 2D gel electrophoresis data", BMC BIOINFORMATICS, BIOMED CENTRAL, LONDON, GB, Bd. 14, Nr. 1, 6. Juli 2013 (2013-07-06), Seiten 215-219, XP021154453, ISSN: 1471-2105, DOI: 10.1186/1471-2105-14-215

## Beschreibung

### Gebiet der Erfindung

Die Erfindung betrifft ein Verfahren zum Überwachen der Probenvorbereitung, Probenreinigung und/oder Probenauftrennung biologischer Proben sowie von chemischen Markierungsreaktionen und Antikörperreaktionen in der biochemischen Analytik und der medizinischen Diagnostik mittels prä-markierter Kontrollproteine. Das erfindungsgemäße Verfahren umfasst insbesondere einen Schritt der Normalisierung der gemessenen Konzentrationen von Zielproteinen anhand der bekannten Konzentration prä-markierter Kontrollproteine. Die Erfindung betrifft weiterhin die Verwendung des Verfahrens zur Qualitätskontrolle in der biochemischen Analytik und der medizinischen Diagnostik.

### Hintergrund der Erfindung

Proteinreinigungen, 1D-Gelelektrophoresen und Western Blot-Analysen (WB) sind neben und in Kombination mit massenspektrometrischen Analysen die häufigsten Werkzeuge für die Untersuchung interessanter Proteine (sogenannter Zielproteine, auch Targetproteine genannt) auf dem Gebiet der Proteinanalytik.

### Proteinreinigungen

Die Zielproteine sind immer Teil komplexer Proteinmischungen (Zell-Lysate, Serum oder andere Proteinrohextrakte). Für die Analyse von Zielproteinen ist es somit nötig, den gewonnenen Proteinrohextrakt zu reinigen, zu konzentrieren oder auf andere herkömmliche Weise zu behandeln. Dabei kommt es häufig zu Proteinverlusten, z.B. durch ungenügendes Fällen oder Resolubilisieren einer Proteinprobe. Dies kann durch die Bestimmung der Gesamtproteinkonzentration oder durch Auftrennung, anschließende Gelelektrophorese und Visualisierung der Proteine und Quantifizierung der Proteinbanden überprüft werden. Die Konzentrationsbestimmung von Gesamtprotein ist häufig nur wenig exakt. Die Färbung von Proteingelen ist zudem sehr aufwändig, schlecht reproduzierbar und die Proteine sind nicht immer quantifizierbar, was eine standardisierbare, objektive Auswertung unmöglich macht. Da Gelelektrophoresen aber fast immer Teil einer Proteinanalyse sind, wäre eine einfach detektierbare, schnelle und quantitative Kontrolle von Proteinverlusten (vorzugsweise während der Probenvorbereitung) direkt auf dem angefertigten Gel ideal. Durch einfache Überwachung und Kontrolle könnten Reinigungen und andere Schritte der Probenvorbereitung besser optimiert werden.

### 1D-Gelelektrophoresen

Während der Gelelektrophorese werden komplexe Proteingemische nach bestimmten Parametern in einer Gelmatrix (meist basierend auf Polyacrylamid oder Agarose) aufgetrennt und anschließend sichtbar gemacht. Die Trennung kann dabei unter denaturierenden oder nativen Bedingungen erfolgen, beispielsweise nach Molekulargewicht (denaturierend), Tertiärstruktur und/oder Größe (nativ) oder isoelektrischem Punkt (nativ oder denaturierend). Zur Visualisierung können unterschiedliche Techniken genutzt werden. Am häufigsten erfolgen post-elektrophoretische Färbungen mit u.a. Silbernitrat, Coomassie®-Brilliantblau oder Fluoreszenzfarbstoffen. Post-elektrophoretische Färbungen haben diverse Nachteile. Sie sind äußerst zeitaufwändig, z.T. wenig sensitiv, wenig reproduzierbar und nicht quantifizierbar. Der größte Nachteil ist aber, dass das Gel für weitere Anwendungen wie Western-Blot Analysen nicht mehr genutzt werden kann, da die Proteine denaturiert und in der Gelmatrix fixiert werden müssen. Eine einfache Möglichkeit, dies zu umgehen, bietet die Markierung von Gesamtprotein mit einem Marker, wie zum Beispiel einem Farbstoff, vorzugsweise einem Fluoreszenzfarbstoff, bereits vor der Gelelektrophorese. Der Fluoreszenz-Marker beispielsweise bietet eine schnelle, unkomplizierte und hochsensitive Möglichkeit zur Visualisierung von Proteinen. Die Markierungsreaktion basiert auf einer chemischen Reaktion der entsprechend aktivierten Fluoreszenzfarbstoffe und den Aminosäure-Resten der Proteine. Dabei können verschiedene Aktivierungen (z.B. NHS-Ester oder Maleimide) und funktionelle Gruppen von Aminosäuren (z.B. Amino-Gruppe von Lysin-Resten oder Thiol-Gruppen von Cystein-Resten) genutzt werden. Wie bei jeder anderen chemischen Reaktionen, kann auch die Effizienz bzw. Ausbeute der Markierung mit Fluoreszenzfarbstoffen durch verschiedene Faktoren sowohl positiv als auch negativ beeinflusst werden. Die Markierung von Lysin-Resten mit aktivierten NHS-Estern wird z.B. durch einen niedrigen pH-Wert oder die Anwesenheit von Aminen inhibiert. Da die Visualisierung von Proteinen die Grundlage für deren Quantifizierung und die weitere Verwendung der Proteinprobe ist, ist es notwendig, dass diese Visualisierung, also die Markierung mit entsprechenden Markern, insbesondere Fluoreszenzfarbstoffen gleichmäßig effizient verläuft. Eine Kontrolle der Effizienz der Markierung ist somit unerlässlich. Des Weiteren ist es für nachfolgende Quantifizierungen ebenso entscheidend, eine gleichmäßige Beladung des Gels mit einer entsprechenden Kontrolle sicherzustellen.

Ein weiteres Problem, das während der 1D Gelelektrophorese auftreten kann, sind gel-interne Laufunterschiede bzw. unterschiedlich große Trennstrecken für verschiedene Proben (z.B. auf Grund von ungleichmäßig polymerisierter Gelmatrix oder ungleichmäßiger/unzureichender Kühlung während der Elektrophorese). Die Laufweite der Proteine bzw. die Trennstrecke ist proportional zum verwendeten Trennparameter, z.B. bei der Standard 1D-SDS-PAGE die Proteingröße. Gerade in routine-diagnostischen Verfahren können bereits kleinste Laufunterschiede das Ergebnis verfälschen, da möglicherweise Proteine auf Grund einer falschen Größenzuordnung nicht mehr oder als falsch erkannt werden. Die Kontrolle der Trennstrecke bzw. Laufweite innerhalb eines Gels kann für jede Probe von entscheidender Bedeutung sein.

### Western-Blot Analysen

Western-Blot Analysen dienen dem spezifischen Nachweis von einem oder mehreren Zielproteinen in einer biologischen Probe, die in der Regel ein komplexes Proteingemisch (Zell-Lysat, Rohextrakt usw.) darstellt, mit Hilfe spezifischer Antikörper-Reaktionen. Komplexe Proteingemische werden durch 1D-Gelelektrophoresen unter unterschiedlich möglichen Bedingungen aufgetrennt bzw. fraktioniert, wie oben beschrieben. Anschließend erfolgt der Transfer der Proteine auf eine Blotting-Membran (meist PVDF oder Nitrocellulose). Auch Membran-freie Western-Blot Analysen (sogenannte In-Gel Western-Blots) sind möglich, kommen jedoch nur selten zur Anwendung. Nach dem Transfer der Proteine von den Elektrophoresegelen auf die Blotting-Membranen erfolgt der immunologische Nachweis des oder der Zielproteine durch die Reaktion mit einem spezifisch gegen das oder die Zielproteine gerichteten Antikörper (AK). Um die Sensitivität dieses immunologischen Nachweises zu erhöhen und mögliche Hintergrundsignale zu minimieren, kommt meist noch ein zweiter Antikörper, der spezifisch den ersten verwendeten Antikörper erkennt und bindet, zum Einsatz. Für die Visualisierung des Zielproteins bzw. der gebundenen Antikörper wird der zuletzt eingesetzte Antikörper mit einem detektierbaren Label konjugiert. Die Konjugation kann zum einem mit einem Enzym erfolgen, das eine sichtbare Farb- oder Lichtreaktion katalysiert oder zum anderen mit einem Fluoreszenzfarstoff, der direkt durch Fluoreszenz-Imaging nachgewiesen werden kann. Im Anschluss erfolgt meist ein weiterer immunologischer Nachweis mit einem Antikörper spezifisch gegen ein sogenanntes House keeping Protein zur Normalisierung des Targetprotein-Signals und/oder eine Färbung des Gesamtproteins auf der Membran, wie z.B. mit Amido Black oder Säureviolett. Dies bedeutet allerdings, dass eine Kontrolle des Erfolgs von Western-Blot Analysen erst ganz am Ende einer solchen Analyse erfolgen kann. War z.B. der Transfer des Proteins auf die Blotting-Membran gestört, wird dies erst sichtbar durch die finale Färbung der Blotting-Membran. Da besonders die Beschaffung von Antikörpern hohe Kosten verursachen und Western-Blot Analysen im Allgemeinen sehr zeitaufwändig sind, wäre eine Kontrolle vor der Behandlung mit den Antikörpern bzw. der einzelnen Teilschritte einer Western-Blot Analyse ökonomisch äußerst sinnvoll. Folgende Kontrollen wären zum Beispiel wünschenswert: (i) Kontrolle der gleichmäßigen Beladung, (ii) Kontrolle eines gleichmäßigen Proteintransfers auf die Blotting-Membran, (iii) Kontrolle der Reaktion des primären Antikörpers und (iv) Kontrolle der Reaktion des sekundären Antikörpers, wobei die Punkte (i) und (ii) einfach durch eine Markierung von Gesamtprotein mit einem Marker, insbesondere einem Fluoreszenzfarbstoff, vor der Gelelektrophorese abgesichert werden können. Dies beinhaltet jedoch den bereits erläuterten Nachteil der fehlenden Kontrolle der Markierung.

### Gel-zu-Gel-Vergleiche

Wenn es häufig die gleichen Zielproteine zu analysieren gilt z.B. bestimmte Proteinbiomarker unter verschiedenen Bedingungen (gesund vs. krank usw.), geschieht dies zwangsläufig auf mehreren Gelen bzw. Western-Blots und z.T. auch durch mehrere Laborkräfte. Bei lange andauernden klinischen Studien werden die Analysen zudem nicht nur auf mehreren Gelen und Western-Blots und verschiedenen Laborkräften durchgeführt, sondern über längere Zeiträume hinweg, wie Wochen, Monate oder Jahre. Schwankungen bei der Durchführung entsprechender Versuche oder möglicherweise auch bei den für die Analysen verwenden Laborchemikalien können nicht ausgeschlossen werden. Somit ist eine Normalisierung des Zielprotein-Signals über verschiedene Gele und Analysen essentiell. Eine Kontrolle, die möglichst Proben-unabhängig eine einfache Möglichkeit zur Normalisierung verschiedener Gele bzw. Western-Blots bietet, ist dabei unerlässlich.

### Zusammenfassung der Erfindung

Die Aufgabe der Erfindung besteht deshalb darin, die zuvor genannten Nachteile des Standes der Technik zu überwinden und eine standardisierbare, objektive Analytik zu ermöglichen. Zur Lösung dieser Aufgabe bietet die vorliegende Erfindung eine neue, unkomplizierte Möglichkeit, eine möglichst große Vielzahl von Teilschritten einer Proteinanalyse zu kontrollieren und zu standardisieren und so deren ökonomische und ökologische Optimierung zu fördern. Die Erfindung beruht auf der exogenen Zugabe von wenigstens einem markierten (prä-markierten) Kontrollprotein zu einer Protein-haltigen Probe (beispielsweise Zell-Lysat, Serum, Proteinrohextrakt usw.). . Dadurch wird
- die quantitative Kontrolle und/oder Visualisierung von Proteinverlusten während der Probenvorbereitung und/oder Proteinreinigung und/oder Gelbeladung,
- die quantitative Kontrolle der Effizienz chemischer Markierungsreaktionen zur Sichtbarmachung der Proteine (z.B. mit Fluoreszenz-Farbstoffen),
- eine absolute Quantifizierung des Gehaltes eines oder mehrerer Ziel-Proteine (innerhalb einer Probe; intra-Assay),
- die Kontrolle und Standardisierung des Laufverhaltens/der Trennstrecke verschiedener Proben innerhalb eines Gels sowie auch Gel-übergreifend,
- Gel-zu-Gel-Vergleiche und/oder -Normalisierungen, und/oder
- die quantitative Kontrolle von AK-Reaktionen bei WB Analysen möglich.

Die Erfindung stellt ein Verfahren zum Nachweis von wenigstens einem Zielprotein in einer Probe bereit, die Proteine enthält, wobei das Verfahren einen Schritt der Markierung der Zielproteine und des prä-markierten Kontrollproteins, und einen Schritt der Normalisierung der quantifizierten Menge und/oder Konzentration von wenigstens einem nachgewiesenen Zielprotein anhand der bekannten Menge und/oder Konzentration des doppelt markierten Kontrollproteins in der Probe umfasst.

Das erfindungsgemäße Verfahren weist insofern mehrere Vorteile auf. Bei Gelelektrophoresen können Proteinverluste einfach, schnell und sogar quantitativ direkt auf dem angefertigten Gel nachgewiesen werden. Dadurch können Reinigungen und andere Schritte der Probenvorbereitung besser optimiert werden. Die nachfolgende Visualisierung der in der Gelelektrophorese aufgetrennten Proteine bildet die Grundlage für deren Quantifizierung und die weitere Verwendung der Proteinprobe. Mit Hilfe des erfindungsgemäßen Verfahrens und Kits ist es möglich, die Effizienz der für die Visulisierung notwendigen Markierungsreaktion zu kontrollieren. Damit kann sichergestellt werden, dass die Markierung mit beispielsweise Fluoreszenzfarbstoffen gleichmäßig effizient verläuft. Des Weiteren kann eine gleichmäßige Beladung des Gels mit einer entsprechenden Kontrolle sichergestellt werden, was für nachfolgende Quantifizierungen entscheidend ist. Außerdem kann das Laufverhalten und die Trennstrecke der einzelnen Proteinproben (auch Gel-übergreifend) kontrolliert und standardisiert werden, was für routine-diagnostische Zwecke entscheidend ist.

Im Falle von Western-Blot Analysen ist es mit dem erfindungsgemäßen Verfahren möglich, beispielsweise (i) die gleichmäßigen Beladung des für den Blot verwendeten Gels und/oder (ii) einen gleichmäßigen Proteintransfers auf die Blotting-Membran und/oder (iii) ein gleichmäßiges Laufverhalten/Trennstrecke der vorangegangen Gelelektrophorese zu kontrollieren. Dabei handelt es sich um die ersten Schritte bei der Anfertigung von Western-Blots. Das erfindungsgemäße Verfahren ermöglicht es also, den Erfolg von Western-Blot Analysen bereits frühzeitig, und nicht wie bisher nur möglich, erst ganz am Ende zu kontrollieren. Frühzeitig heißt, dass mit dem erfindungsgemäßen Verfahren die Kontrollen bereits vor Behandlung mit den notwendigen in der Regel teuren Antikörpern erfolgen können. Bei festgestellten Störungen in der Gelelektrophorese und/oder beim Proteintransfer auf die Blotting-Membran können die gestörten Analysen frühzeitig abgebrochen werden, was zur Einsparung von Zeit, Kosten und Ressourcen führt.

Das erfindungsgemäße Verfahren stellt weiterhin eine einfache und Proben-unabhängig Möglichkeit für die Normalisierung des Zielprotein-Signals über verschiedene Gele und Analysen bereit. Dies ist besonders wichtig bei der Analyse sehr hoher Probenzahlen und/oder wenn viele Proben, beispielsweise während der Durchführung von klinischen Studien, über einen längeren Zeitraum gemessen werden sollen.

In einer weiteren Ausführungsform betrifft die Erfindung die Verwendung des Verfahrens. Das Verfahren dient der Qualitätskontrolle und der Verbesserung der Patientensicherheit. Durch die Einführung des positiven Kontrollproteins können sowohl Schwankungen bei der Quantifizierung und Zuordnung eines Zielproteins in verschiedenen Proben einer Messung/Messreihe, aber auch zwischen den Proben verschiedener Messungen, die beispielsweise an zwei unterschiedlichen Zeitpunkten vorgenommen worden sind, ermittelt und durch den Normalisierungsschritt ausgeräumt werden. Durch die Einführung des markierten Kontrollproteins ist es weiterhin möglich, falsch-positive bzw. falsch-negative Messergebnisse auszuschließen.

### Detaillierte Beschreibung der Erfindung

Die Erfindung beruht auf der exogenen Zugabe mindestens eines prä-markierten Kontrollproteins zu einer Protein-haltigen Probe, um möglichst viele Teilschritte einer Proteinanalyse quantitativ kontrollieren zu können. Ein nicht zur analysierenden Proteinprobe gehörendes Kontrollprotein wird mit einem einfach zu detektierenden Marker markiert. Dieser Marker ist vorzugsweise ein Fluoreszenzfarbstoff, da dieser durch einfaches Fluoreszenz-Imaging visualisiert werden kann.

Nach der Markierung werden die markierten Proteine und/oder Proteinfragmente einer Trennung, Detektion und Analyse unter Verwendung herkömmlicher Verfahren, wie beispielsweise Elektrophorese-Verfahren, chromatographischen Verfahren, Immunblotting, Massenspektroskopie und Kombinationen davon unterzogen. Solche Verfahren können zur Markierung und Analyse von Protein-Proben, beispielsweise Proben von sauren Proteinen, basischen Proteinen, gereinigten Proteinen oder Lysaten verwendet werden. Die Verfahren sind hochsensitiv und haben einen breiten linearen Detektionsbereich. Die Verfahren gemäß der Erfindung sind reproduzierbar und schnell.

Die Erfindung stellt ein Verfahren zum Überwachen der Probenvorbereitung, Probenreinigung und/oder Probenauftrennung biologischer Proben sowie von chemischen Markierungsreaktionen und Antikörperreaktionen in einer Probe, die Proteine enthält, bereit, wobei das Verfahren die Schritte umfasst:
i. Zugabe eines Kontrollproteins zu der Probe, wobei das Kontrollprotein vor Zugabe zu der Probe markiert wurde,
ii. Markierung des wenigsten einen Zielproteins und des markierten Kontrollproteins, vorzugsweise mit einem Fluoreszenz-Farbstoff, in der Probe,
iii. Auftrennung der Proteine in der Probe mittels Gelelektrophorese zur Separation des wenigstens einen markierten Zielproteins und des doppelt-markierten Kontrollproteins,
iv. Nachweis und/oder Quantifizieren des wenigstens eines Zielproteins und des markierten Kontrollproteins,
v. Normalisierung der quantifizierten Menge und/oder Konzentration und/oder des Laufverhaltens/der Trennstrecke und/oder absolute Quantifizierung des wenigstens einen Zielproteins anhand der bekannten Menge und/oder Konzentration und/oder des Laufverhaltens/der Trennstrecke des markierten Kontrollproteins in der Probe.

In einer Ausführungsform eignet sich das erfindungsgemäße Verfahren für die Kontrolle der Probenvorbereitung. Durch Zugabe einer definierten Menge von prä-markiertem Kontrollprotein zu einer Proteinprobe kann beispielsweise die anschließende Probenvorbereitung/-aufreinigung dieser Probe kontrolliert werden. Während der Probenvorbereitung kann es zu Verlusten an Protein kommen, z.B. durch unvollständige Proteinfällungen, ungleichmäßige Gelbeladung oder auch simple Pipettierfehler. Der Verlust von zu analysierendem Protein korreliert mit dem Verlust von markiertem Kontrollprotein. Die verbleibende Menge an markiertem Kontrollprotein kann nach der Analyse durch die Prä-Markierung, vorzugsweise Fluoreszenzmarkierung, einfach und schnell detektiert werden. Unterschiede in den Mengen an verbliebenem Kontrollprotein können verwendet werden, um die während der Analyse nachgewiesenen Mengen verschiedener Zielproteine in der Probe rechnerisch auszugleichen.

In einer bevorzugten Ausführungsform des erfindungsgemäßen Verfahrens wird das wenigstens ein Zielprotein mit einem Fluoreszenzfarbstoff markiert.

Proben, die in einem Verfahren der Erfindung analysiert werden können, schließen Vollblut, Blutserum oder Plasma, Urin, Speichel, Hirnflüssigkeit (CSF) oder andere Körperflüssigkeiten (Stuhl, Tränenflüssigkeit, Gelenkflüssigkeit, Sputum), Atem (z.B. als kondensierter Atem, oder ein Extrakt oder eine Aufreinigung oder eine Verdünnung davon) ein. Biologische Proben schließen auch Gewebehomogenate, Gewebesektionen und Biopsien von einem lebenden Probanden oder post-mortem entnommene ein. Die Proben können auf herkömmliche Weise vorbereitet und aufbewahrt werden, zum Beispiel, wo angebracht, verdünnt oder konzentriert werden.

Die Kontrollproteine können verschieden oder auch identisch mit Proteinen in der Proteinprobe sein. In einer bevorzugten Ausführungsform der Erfindung sind die Kontrollproteine verschieden von den Zielproteinen. In einer weiteren bevorzugten Ausführungsform sind die Kontrollproteine mit den Zielproteinen identisch.

Das erfindungsgemäße Verfahren ist insbesondere dadurch kennzeichnet, dass die Kontrollproteine von extern zu der Probe zugegeben werden, d.h. die Kontrollproteine sind bei Entnahme und/oder Erzeugung der Proben nicht schon in der Probe enthalten.

Die Kontrollproteine werden vor Zugabe zu der Probe markiert.

Grundsätzlich sind alle herkömmlichen Marker zur Markierung der Zielproteine und der Kontrollproteine für die Verwendung in dem erfindungsgemäßen Verfahren geeignet. Bevorzugt für die Markierung sind jedoch Fluoreszenz-Farbstoffe, da diese ein einfaches und schnelles Auslesen ermöglichen.

In einer besonders bevorzugten Ausführungsform der Erfindung erfolgt die Markierung der Zielproteine und der Kontrollproteine mit unterschiedlichen Farbstoffen, vorzugsweise mit Fluoreszenzfarbstoffen unterschiedlicher Farbe. Besonders geeignet sind Fluoreszenzfarbstoffe unterschiedlicher Farbe, die unterschiedliche Absorptions- und/oder Emissionsmaxima aufweisen. Ganz besonders geeignet für die Verwendung in dem erfindungsgemäßen Verfahren sind Fluoreszenzfarbstoffe, deren Fluoreszenzanregungs- und Emissionsmaxima im UV- bis IR-Bereich liegen. Die Wellenlänge der Anregungsmaxima der Fluoreszenzfarbstoffe gemäß der Erfindung liegt vorzugsweise im Bereich von 495 nm bis 740 nm. Die Wellenlänge der Emissionsmaxima der Fluoreszenzfarbstoffe gemäß der Erfindung liegt vorzugsweise im Bereich von 525 nm bis 765 nm.

Das oder die markierten Kontrollproteine werden in dem erfindungsgemäßen Verfahren vor der Markierung der Zielproteine zu der Probe gegeben. Wenn das markierte Kontrollprotein vor der Markierung der Zielproteine zu der Probe gegeben wird, wird das markierte Kontrollprotein durch den sich anschließenden Markierungsschritt des wenigstens einen Zielproteins nochmals markiert, also doppelt markiert. Idealerweise ist das Kontrollprotein dann mit zwei verschiedenen Farbstoffen, vorzugsweise Fluoreszenzfarbstoffen, doppelt markiert. Diese doppelte Markierung ist besonders nützlich für das Überwachen der Markierungsreaktion der Zielproteine.

In dem erfindungsgemäßen Verfahren können einzelne oder mehrere verschiedene Kontrollproteine eingesetzt werden. Verschiedene Kontrollproteine können vor Zugabe zur Proteinprobe gemischt werden. Die Visualisierung der Kontrollen ist entweder direkt im Anschluss an die Gelelektrophorese, während oder nach anschließenden Analysen (z.B. Western-Blot Analysen) möglich.

Die Kontrollproteine können ein Molekulargewicht im Bereich von 0,5 bis 500 kDa, vorzugsweise 2,0 bis 400 kDa, besonders bevorzugt 3,0 bis 300 kDa haben. In einer ganz besonders bevorzugten Ausführungsform haben die Kontrollproteine ein Molekulargewicht von mindestens 5 kDa aber höchstens 250 kDa. Das verwendete Molekulargewicht ist dabei abhängig von der Zusammensetzung und Beschaffenheit der Proteinprobe und dem eigentlichen Zielprotein der Analyse. Die Kontrollproteine können natürlich vorkommend, rekombinant oder künstlich hergestellt sein.

Kontrollproteine im Sinne der Erfindung können auch Peptide, insbesondere synthetisch hergestellt Peptide, sein und haben vorzugsweise 5 bis 5000 Aminosäuren, vorzugsweise 20 bis 4000 Aminosäuren, besonders bevorzugt 30 bis 3000 Aminosäuren. In einer ganz besonders bevorzugten Ausführungsform haben die Kontrollproteine mindestens 50 Aminosäuren, höchstens aber 2500 Aminosäuren.

In einer weiteren bevorzugten Ausführungsform der Erfindung sind die Kontrollproteine rekombinante oder synthetisch hergestellte Proteine mit einem Molekulargewicht von mindestens 5 kDa aber höchstens 250 kDa bzw. mit 50 bis 2500 Aminosäuren. Besonders bevorzugt sind rekombinante oder synthetisch hergestellt Kontrollproteine mit einem Molekulargewicht von 10 kDa bis 150 kDa bzw. mit 100 bis 1500 Aminosäuren. In einer weiteren bevorzugten Ausführungsform sind die Kontrollproteine reich an Aminosäuren, die die Anbindung der Marker, insbesondere der Fluoreszenzfarbstoffe ermöglichen. Besonders bevorzugt sind Kontrollproteine, die ein oder mehrere Lysin- und/oder Cystein-Reste aufweisen.

Alle Fluoreszenzfarbstoffe, die an Proteine binden können, sind prinzipiell zur Anwendung in dem erfindungsgemäßen Verfahren geeignet. Die Fluoreszenzfarbstoffe können z.B. ausgewählt sein aus den Gruppen der Phycobiline, Rhodamine und Safranine. Geeignete Fluoreszenzfarbstoffe können z.B. ausgewählt sein aus der Gruppe, die aus Acridingelb, Acridinorange, Aequorin, Aesculin, Allophycocyanin, 7-Aminoactinomycin, Auramin O, Berberin, 9,10-Bis(phenylethinyl)anthracen, Calcein, 6-Carboxyfluorescein, Chinin, CumarinFarbstoffe, Cyaninen, 4',6-Diamidin-2-phenylindol, 2,7-Dichlorfluorescein, 9,10-Diphenylanthracen, Eosin B, Eosin Y, Epicocconon, Ethidiumbromid, 6-FAMphosphoramidit, Flavinen, Fluoren, Fluorescein, Fluorescein Arsen Helix Bindern, Fura-2, Furaptra, Grün fluoreszierendes Protein (GFP), Hoechst 33342, IAEDANS, Indischgelb, Indocyaningrün, Luciferinen, Merbromin, N-Methylacridon, Nilblau, Nilrot, Phycocyanin, Phycoerythrin, Propidiumiodid, Pyranin, Rhodamin B, Rubren, Safranin T, Stilben, SYBR Green I, SYPRO Orange, SYPRO Red, SYPRO Ruby, Texas Red, TMRM+, Umbelliferon und Xylenolorange besteht.

Die Fluoreszenzfarbstoffe sind vorzugsweise kovalent an die Zielproteine bzw. die Kontrollproteine gebunden. Besonders bevorzugt ist es, wenn die Bindung und/oder Aktivierung der Fluoreszenzfarbstoffe über NHS-Ester, Maleimide, funktionelle Gruppen von Aminosäuren (z.B. Amino-Gruppe von Lysin-Resten oder Thiol-Gruppen von Cystein-Resten) erfolgt.

Es sind verschiedene Techniken zur Trennung bzw. Aufreinigung von Proteinen bekannt. Für die Durchführung des erfindungsgemäßen Verfahrens werden Gelelektrophoreseverfahren verwendet. Grundsätzlich können alle herkömmlichen Gelelektrophoreseverfahren in dem erfindungsgemäßen Verfahren eingesetzt werden. Herkömmliche Gelelektrophoreseverfahren sind zum Beispiel SDS-PAGE (sodium dodecyl sulfate polyacrylamide gel electrophoresis, Natriumdodecylsulfat-Polyacrylamidgelelektrophorese) zur Trennung von Stoffgemischen (häufig Proteinen) nach Molekülgröße, IEF (isoelektrische Fokussierung) zur Trennung von Proteinen nach ihrem isoelektrischem Punkt, 2D-Gelelektrophorese als Kombination aus SDS-Page und IEF für komplexe Proteingemische, QPNC-PAGE (QPNC: quantitative preparative native continuous polyacrylamide gel electrophoresis) zur Analytik von Metalloproteinen, Nativ-Gelelektrophorese zur Untersuchung der Proteinfaltung, SDD-AGE (semi-denaturing detergent agarose gel electrophoresis, halbdenaturierende detergenzvermittelte Agarose-Gelelektrophorese) zur Untersuchung von Proteinaggregaten, Pulsed-Field-Gelelektrophorese (PFGE) und Kapillarelektrophorese (CE).

Besonders bevorzugt gemäß der Erfindung sind alle 1D-Gelelektrophoreseverfahren, wie z.B. SDS-PAGE, IEF, QPNC-PAGE, Nativ-Gelelektrophorese zur Untersuchung der Proteinfaltung, SDD-AGE und PFGE.

Nach der Auftrennung der Proteinprobe in der Gelelektrophorese können die Elektrophoresegele unmittelbar dazu genutzt werden, um die Zielproteine und Kontrollproteine, wie beispielsweise deren Menge oder Konzentration, mittels eines geeigneten Detektionssystems sofort zu analysieren. Bereits hier kann festgestellt werden, ob die Markierungsreaktion zur Markierung der Zielproteine und der Kontrollproteine erfolgreich war und ob die Gelelektrophorese störungsfrei verlaufen ist. Wenn nach der Auftrennung der Probe mittels Gelelektrophorese ein Immunoblot, wie z.B. ein Western-Blot, durchgeführt wird, kann die Blotting-Membran zur Detektion und Auswertung der Fluoreszenzsignale der Zielproteine und Kontrollproteine herangezogen werden bzw. zusätzlich zu einer bereits vorher erfolgten Detektion und Auswertung der Elektrophoresegele. Mit der Detektion und Auswertung der Fluoreszenzsignale der Zielproteine und Kontrollproteine nach erfolgtem Western-Blot ist es möglich, neben der Überwachung und Kontrolle der Markierungsreaktion und des Elektrophoreseschrittes auch den Schritt der Durchführung des Western-Blots zu überwachen und zu kontrollieren.

Das oder die markierten Kontrollproteine werden vor dem Auftragen auf das Elektrophoresegel direkt zur Probe zugegeben.

Nach Durchlaufen der Elektrophorese und/oder im Anschluss an den Western-Blot erfolgt die Normalisierung der Menge und/oder Konzentration und/oder des Laufverhaltens/der Trennstrecke wenigstens eines Zielproteins anhand der bekannten Menge und/oder Konzentration und/oder des Laufverhaltens/ der Trennstrecke wenigstens eines Kontrollproteins.

Das erfindungsgemäße Verfahren ist besonders geeignet für die Normalisierung der quantifizierten Menge des wenigstens einen Zielproteins anhand der bekannten Menge des markierten Kontrollproteins in der Probe. Das erfindungsgemäße Verfahren eignet sich auch für die die Normalisierung der Konzentration des wenigstens einen Zielproteins anhand der bekannten Konzentration des markierten Kontrollproteins in der Probe. In einer weiteren Ausführungsform eignet sich das erfindungsgemäße Verfahren auch zur absolute Quantifizierung des wenigstens einen Zielproteins anhand der bekannten Menge und/oder Konzentration des markierten Kontrollproteins in der Probe.

Vorzugsweise erfolgt die Normalisierung von Zielproteinsignalen auf einem Gel oder einer Blotting-Membran nach der Elektrophorese und/oder dem Western-Blot gegen die Signale des oder der markierten Kontrollproteine (Verhältnis zwischen Zielprotein/Kontrollprotein).

Die Normalisierung kann dabei zwischen Ziel- und Kontrollproteinen erfolgen, die auf einem Elektrophoresegel und/oder einer Blotting-Membran aufgetragen worden sind. Dabei handelt es sich um eine sogenannte "intra-Assay-Normalisierung". Mit der intra-Assay-Normalisierung können Störungen bei der Markierungsreaktion der Zielproteine, bei der Gelelektrophorese und/oder beim Western-Blot frühzeitig nachgewiesen werden.

Die Normalisierung kann aber auch zwischen, beispielsweise der Menge und/oder Konzentration wenigstens eines Zielproteins und/oder wenigstens eines Kontrollproteins, die auf ein erstes Elektrophoresegel aufgetragen worden sind, und der bekannten Menge und/oder Konzentration wenigstens eines Kontrollproteins, das auf ein zweites Elektrophoresegel aufgetragen worden ist, erfolgen. In gleicher Weise kann die Normalisierung auch zwischen, beispielsweise der Menge und/oder Konzentration wenigstens eines Zielproteins und/oder wenigstens eines Kontrollproteins, die auf eine erste Blotting-Membran aufgetragen worden sind, und der bekannten Menge und/oder Konzentration wenigstens eines Kontrollproteins, das auf eine zweite Blotting-Membran aufgetragen worden ist, erfolgen. Dabei handelt es sich um eine sogenannte "inter-Assay-Normalisierung". Die inter-Assay-Normalisierung dient vor allem der Qualitätskontrolle bei der Analyse größerer Probenserien, die entweder sehr hohe Probenzahlen umfassen und/oder über längere Zeiträume, wie mehrere Tage, Wochen, Monate oder Jahre durchgeführt werden, um so auftretende Schwankungen der Assay-Parameter (wie z.B. unterschiedliche Imaging-Parameter wie Belichtungszeiten) auszugleichen.

Die Normalisierung erfolgt vorzugsweise anhand des Signals, besonders bevorzugt anhand des Fluoreszenzsignals des wenigstens einen markierten Kontrollproteins in der Probe. Beispielsweise wird das Signal des Kontrollproteins in einer ersten Probe gleich 1 gesetzt. Die Signale des markierten Kontrollproteins in den weiteren Proben, also der zweiten, dritten usw. Probe stehen über einen bestimmten Faktor mit dem aus der ersten Probe im Verhältnis. Dieser Faktor kann verwendet werden, um die Signale des Zielproteins zu normalisieren.

Die Normalisierung kann aber auch auf Basis der Bandenvolumina erfolgen. Dabei können die Signale des Kontrollproteins verwendet werden, um die Signale der Zielproteine zu normalisieren. Beispielsweise kann das Bandenvolumen x des Kontrollproteins in einer ersten Probe mit dem Bandenvolumen y des Kontrollproteins in einer zweiten Probe über den Faktor x/y im Verhältnis stehen. Dieser Faktor kann dann verwendet werden, um die Bandenvolumina Proben-interner Proteine zu normalisieren.

In einer besonders bevorzugten Ausführungsform der Erfindung beruht die Normalisierung darauf, dass die Signale und/oder die Bandenvolumina des markierten Kontrollproteins in einer ersten Proben und wenigstens einer weiteren Probe miteinander in ein Verhältnis gesetzt werden und das so ermittelte Verhältnis angewendet wird, um die Menge und/oder Konzentration des Zielproteins zu bestimmen und/oder zu quantifizieren. Das erfindungsgemäße Verfahren hat somit den Vorteil, dass die Bestimmung der korrekten Menge und/oder Konzentration des Zielproteins in einer Probe durch einen sehr einfachen Vergleich mit dem markierten Kontrollprotein in Echtzeit erfolgen kann. Das Molekulargewicht und andere Eigenschaften des Zielproteins müssen nicht bekannt sein. Komplizierte Auswerteverfahren, wie beispielweise das vorherige Erstellen von Eichkurven, die zudem noch für jedes einzelne (Ziel-) Protein spezifisch sind, können entfallen.

Wenn das Laufverhalten/die Trennstrecke verschiedener Proben innerhalb eines Gels kontrolliert werden soll, erfolgt der Schritt der Normalisierung dadurch, dass die Trennstrecke des wenigstens einen markierten Kontrollproteins (Laufweite des Kontrollproteins ab Probenauftrag und/oder der Abstand des markierten Kontrollproteins zu einem weiteren Kontrollprotein) in einer Probe ins Verhältnis gesetzt wird zur Laufweite des Zielproteins und/oder zum Abstand des Zielproteins zum ersten Kontrollprotein und/oder zum Abstand des Zielproteins zum zweiten Kontrollprotein in dieser ersten Probe (intra-Assay). Damit kann beispielsweise ein Zielprotein auf Grund des Laufverhaltens identifiziert werden.

Wenn das Laufverhalten/die Trennstrecke verschiedener Proben zwischen unterschiedlichen Gelen kontrolliert werden soll, erfolgt der Schritt der Normalisierung beispielsweise dadurch, dass die Laufweite des wenigsten einen markierten Kontrollproteins (Strecke zwischen Kontrollprotein und Probenauftrag und/oder einem weiterem Kontrollprotein) in einer Probe ins Verhältnis gesetzt wird zur Laufweite des wenigsten einen Kontrollproteins in wenigsten einer weiteren Probe (inter-Assay). Der für das Kontrollprotein ermittelte Wert für das Verhältnis der Laufweiten kann dann entsprechend zur Normalisierung der Laufweite eines Zielproteins eingesetzt werden. Das hat den Vorteil, dass auch bei starken Unterschieden im Laufverhalten innerhalb eines Gels oder über mehrere Gele hinweg, die Zielproteine eindeutig identifiziert und im Falle von mehreren sich sehr ähnelnden Zielproteinen diese eindeutig diskriminiert werden können.

Die Auswertung, vorzugsweise der Fluoreszenzsignale, kann mit jeder dazu geeigneten herkömmlichen Auswerteeinrichtung erfolgen. Bevorzugt zur Verwendung in dem erfindungsgemäßen Verfahren sind Auswerteeinrichtungen, die über eine Auswertesoftware verfügen und das Verhältnis der Signale und/oder die Bandenvolumina des markierten Kontrollproteins in einer ersten Proben und wenigstens einer weiteren Probe automatisiert ermitteln können. Wahlweise kann das Verhältnis der Signale und/oder die Bandenvolumina des markierten Kontrollproteins in einer ersten Probe und wenigstens einer weiteren Probe auch manuell ermittelt werden. Für die Durchführung des erfindungsgemäßen Verfahrens kann also die herkömmliche bereits zur Laborausstattung gehörende Ausrüstung benutzt werden, sofern diese zur Fluoreszenzdetektion geeignet ist. Neuanschaffungen sind nicht nötig.

Die Erfindung stellt also ein schnelles, einfaches und kostengünstiges Verfahren bereit, mit dem alle Schritte der Analyse von Proteinen, die auf Gelelektrophorese beruhen, beginnend von der Probenvorbereitung über die optionale Markierung der Zielproteine, die Durchführung der Gelelektrophorese selbst bis hin zum anschließenden Immunoblotting überwacht werden können.

Die Erfindung betrifft weiterhin die Verwendung des erfindungsgemäßen Verfahrens zum Überwachen der Probenvorbereitung, Probenreinigung und/oder Probenauftrennung proteinhaltiger Proben, zum Überwachen von chemischen Markierungsreaktionen und Antikörperreaktionen und/oder zur Qualitätskontrolle in der biochemischen Analytik und/oder der medizinischen Diagnostik.

In einer bevorzugten Ausführungsform betrifft die Erfindung die Verwendung des erfindungsgemäßen Verfahrens zur
- quantitativen Kontrolle und/oder Visualisierung von Proteinverlusten während der Probenvorbereitung und/oder Proteinreinigung und/oder Gelbeladung,
- quantitativen Kontrolle der Effizienz chemischer Markierungsreaktionen (z.B. mit Fluoreszenz-Farbstoffen),
- absoluten Quantifizierung des Gehaltes eines oder mehrerer Zielproteine innerhalb einer Probe (intra-Asay) oder zwischen verschiedenen Assays (inter-Assay),
- Durchführung von Gel-zu-Gel-Vergleichen und/oder -Normalisierungen (inter-Assay),
- die Kontrolle und Standardisierung des Laufverhaltens/der Trennstrecke verschiedener Proben innerhalb eines Gels sowie Gel-übergreifend,
- zur quantitativen Kontrolle von Antikörper-Reaktionen bei Western-Blott Analysen, und/oder
- zum Ausschluss falsch-positiver bzw. falsch-negativer Messergebnisse.

Die Erfindung ist somit durch die folgende Merkmale gekennzeichnet:
1. Verfahren zum Überwachen der Probenvorbereitung, Probenreinigung und/oder Probenauftrennung biologischer Proben sowie von chemischen Markierungsreaktionen und Antikörperreaktionen in einer Probe, die Proteine enthält, umfassend die Schritte:
   i. Zugabe eines Kontrollproteins zu der Probe, wobei das Kontrollprotein vor Zugabe zu der Probe markiert wurde,
   ii. optional Schritte der Probenvorbereitung (z.B. Proteinfällungen),
   iii. Markierung des wenigsten einen Zielproteins und des Kontrollproteins, vorzugsweise mit einem Fluoreszenz-Farbstoff, in der Probe,
   iv. Auftrennung der Proteine in der Probe mittels Gelektrophorese zur Separation des wenigstens einen markierten Zielproteins und des doppelt-markierten Kontrollproteins,
   v. Nachweis und/oder Quantifizieren des wenigstens eines Zielproteins und des markierten Kontrollproteins,
   vi. Normalisierung der quantifizierten Menge und/oder Konzentration und/oder des Laufverhaltens/der Trennstrecke und/oder absolute Quantifizierung des wenigstens einen Zielproteins anhand der bekannten Menge und/oder Konzentration und/oder des Laufverhaltens/der Trennstrecke des markierten Kontrollproteins in der Probe.
2. Verfahren nach Merkmal 1, wobei das Kontrollprotein das gleiche Protein wie das Zielprotein ist.
3. Verfahren nach Merkmal 1, wobei das Kontrollprotein von dem Zielprotein verschieden ist.
4. Verfahren nach einem der Merkmale 1 bis 3, wobei die Markierung des Zielproteins und des Kontrollproteins mit Farbstoffen, vorzugsweise Fluoreszenzfarbstoffen erfolgt.
5. Verfahren nach einem der Merkmale 1 bis 4, wobei die Fluoreszenzfarbstoffe kovalent an das Zielprotein und das Kontrollprotein gebunden sind.
6. Verfahren nach Merkmal 5, wobei die Bindung und/oder Aktivierung der Fluoreszenzfarbstoffe über NHS-Ester, Maleimide, funktionelle Gruppen von Aminosäuren (z.B. Amino-Gruppe von Lysin-Resten oder Thiol-Gruppen von Cystein-Resten) erfolgt.
7. Verfahren nach einem der Merkmale 1 bis 6, wobei das Kontrollprotein ein natürliches Protein, ein rekombinantes Protein oder ein synthetisch hergestelltes Protein ist.
8. Verfahren nach einem der Merkmale 1 bis 7, wobei die Probe ausgewählt sein kann aus Vollblut, Blutserum oder Plasma, Urin, Speichel, Hirnflüssigkeit (CSF), anderen Körperflüssigkeiten (Stuhl, Tränenflüssigkeit, Gelenkflüssigkeit, Sputum), Atem (z.B. als kondensierter Atem, oder einem Extrakt oder einer Aufreinigung oder einer Verdünnung davon), Gewebehomogenate, Gewebesektionen und Biopsien.
9. Verfahren nach einem der Merkmale 1 bis 9, wobei die Auftrennung der Proteine in der Probe mittels 1D-Gelelektrophorese erfolgt.
10. Verfahren nach einem der Merkmale 1 bis 10, wobei die Auftrennung der Proteine in der Probe mittels SDS-PAGE, IEF QPNC-PAGE, Nativ-Gelelektrophorese, SDD-AGE oder PFGE erfolgt.
11. Verfahren nach einem der Merkmale 9 bis 11, wobei nach der Auftrennung der Proteine mittels Gelelektrophorese ein Western-Blot durchgeführt wird.
12. Verfahren nach einem der Merkmale 1 bis 13, wobei die Normalisierung zwischen wenigstens einem Zielprotein und wenigstens einem Kontrollproteinen erfolgt, die auf dem gleichen Elektrophoresegel und/oder der gleichen Blotting-Membran aufgetragen worden sind (intra-Assay).
13. Verfahren nach einem der Merkmale 1 bis 14, wobei die Normalisierung zwischen wenigstens einem Zielprotein und/oder wenigstens einem Kontrollproteinen erfolgt, die auf verschiedenen Elektrophoresegelen und/oder verschiedenen Blotting-Membranen aufgetragen worden sind (inter-Assay).
14. Verwendung des Verfahrens nach einem der Merkmale 1 bis 15 zum Überwachen der Probenvorbereitung, Probenreinigung und/oder Probenauftrennung proteinhaltiger Proben.
15. Verwendung des Verfahrens nach einem der Merkmale 1 bis 15zum Überwachen von chemischen Markierungsreaktionen und Antikörperreaktionen.
16. Verwendung des Verfahrens nach einem der Merkmale 1 bis 15zur Qualitätskontrolle in der biochemischen Analytik und/oder der medizinischen Diagnostik.
17. Verwendung nach einem der Merkmale 16 bis 18 zur
   - quantitativen Kontrolle und/oder Visualisierung von Proteinverlusten während der Probenvorbereitung und/oder Proteinreinigung und/oder Gelbeladung,
   - quantitativen Kontrolle der Effizienz chemischer Markierungsreaktionen (z.B. mit Fluoreszenz-Farbstoffen),
   - absoluten Quantifizierung des Gehaltes eines oder mehrerer Zielproteine innerhalb einer Probe oder zwischen verschiedenen Assays (intra-Assay),
   - Durchführung von Gel-zu-Gel-Vergleichen und/oder -Normalisierungen,
   - Kontrolle und Standardisierung des Laufverhaltens/der Trennstrecke verschiedener Proben innerhalb eines Gels sowie auch Gel-übergreifend,
   - quantitativen Kontrolle von Antikörper-Reaktionen bei Western-Blott Analysen, und/oder
   - zum Ausschluss falsch-positiver bzw. falsch-negativer Messergebnisse.

Die Erfindung wird nachfolgend anhand von 8 Zeichnungen und 3 Ausführungsbeispielen näher erläutert.
**Figur 1** zeigt die Kontrolle einer Probenvorbereitung.
**Figur 2** zeigt die Kontrolle der Effizienz von Markierungsreaktionen.
**Figur 3** zeigt die Verwendung von Kontrollproteinen zur Quantifizierung.
**Figur 4** zeigt die Verwendung von Kontrollproteinen zur Standardisierung des Laufverhaltens/ der Trennstrecke
**Figur 5** zeigt die Verwendung von Kontrollproteinen zum Gel-zu-Gel-Vergleich (inter-Assay).

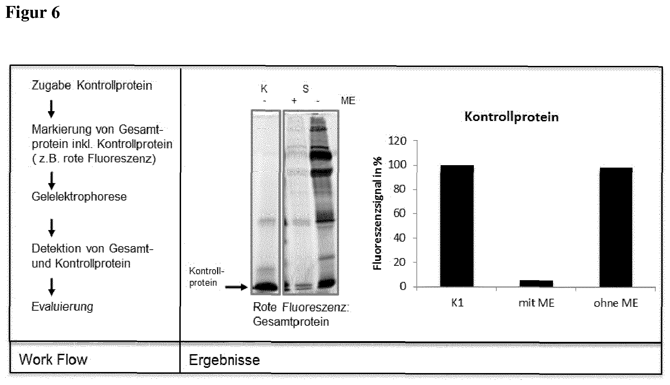

**Figur 6** zeigt die Kontrolle der Effizienz einer Markierungsreaktion. Die schematische Darstellung zeigt den Ablauf des Ablaufs des Versuchs. Fluoreszenzdetektion (FujiFilm FLA-9000) von T-Red (rote Fluoreszenz)-markierten Proteinproben nach Auftrennung durch 1D-SDS-PAGE (links; K: Kontrolle (optimale Markierung des Kontrollproteins); S: humanes Serum; ME: beta-Mercaptoethanol). Bandendetektion und Quantifizierung des Bandenvolumens erfolgten mittels LabImage 1D. Das Bandenvolumen bzw. Fluoreszenz-Signal des Kontrollproteins in der Kontrollprobe wurde gleich 100% gesetzt und die Bandenvolumina des Kontrollproteins in den beiden Serumproben (mit und ohne Zugabe von 1% beta-Mercaptoethanol) wurde dazu ins Verhältnis gesetzt (links).
**Figur 7** zeigt die Kontrolle des Kontrollproteins. Die schematische Darstellung zeigt den Ablauf des Versuchs. Ergebnisse: Fluoreszenzdetektion (FujiFilm FLA-9000) von T-Green (grüne Fluoreszenz)-prä-markiertem Kontrollprotein und T-Red (rote Fluoreszenz)-markierten Proteinproben nach Auftrennung durch 1D-SDS-PAGE und anschließender Silberfärbung (K: Kontrolle; A1 bis A4: humane Proteinproben).

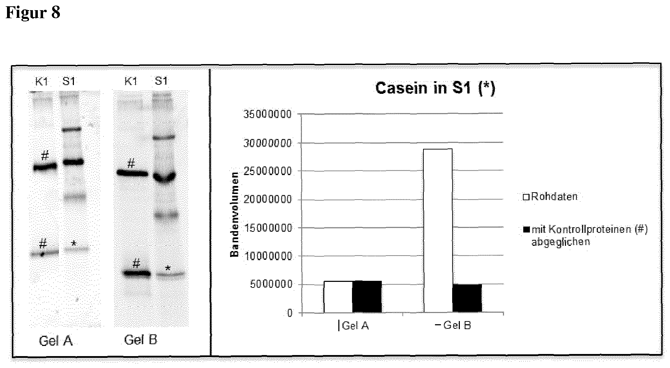

**Figur 8** zeigt die Verwendung von Kontrollproteinen für den Gel-zu-Gel-Vergleich. Links: Fluoreszenzdetektion (FujiFilm FLA-9000) von T-Red (rote Fluoreszenz)-markierten Proteinproben nach Auftrennung durch 1D-SDS-PAGE in zwei verschiedenen Gelen A und B (K: Kontrollproteine BSA und beta-Casein (je 0,5 µg), S1: Standardprotein-Mischung (je 5 µl)). Bandendetektion und Quantifizierung des Bandenvolumens erfolgten mittels LabImage 1D. Rechts: dargestellt sind jeweils die ermittelten Bandenvolumen der beta-Casein Bande (*) in den Gelen A und B vor und nach rechnerischem Abgleich mit dem Kontrollprotein.

### Probenvorbereitung

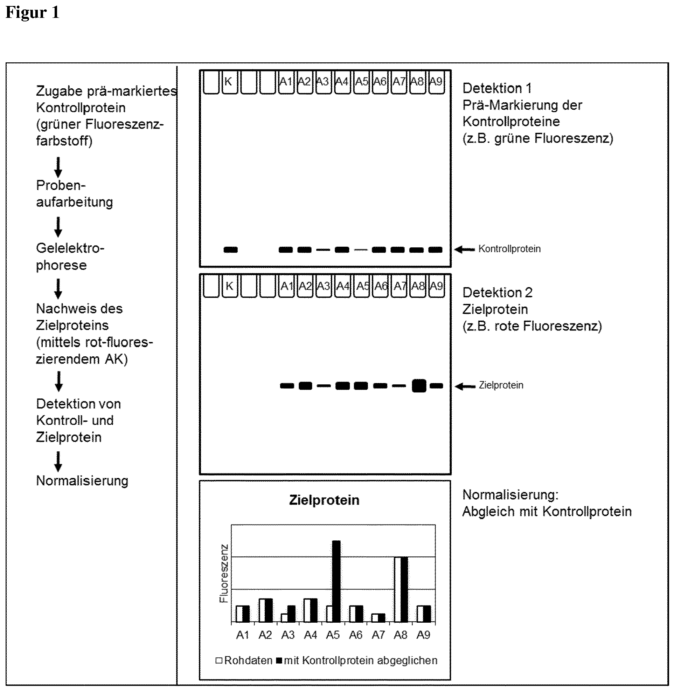

Durch Zugabe einer definierten Menge von prä-markiertem Kontrollprotein zu einer Proteinprobe kann die anschließende Probenvorbereitung/-aufreinigung dieser Probe kontrolliert werden (siehe **Figur 1**). Während der Probenvorbereitung kann es zu Verlusten an Protein kommen, z.B. durch unvollständige Proteinfällungen, ungleichmäßige Gelbeladung oder auch simple Pipettierfehler. Der Verlust von zu analysierendem Protein korreliert mit dem Verlust von Kontrollprotein. Die verbleibende Menge an Kontrollprotein kann nach der Analyse durch die Prä-Markierung einfach und schnell detektiert werden. Unterschiede in den Mengen an verbliebenem Kontrollprotein können verwendet werden, um die während der Analyse nachgewiesenen Mengen verschiedener Zielproteine in der Probe rechnerisch auszugleichen. Beispiel: Das Signal des Kontrollproteins in Probe A1 wird gleich 1 gesetzt. Die Signale des Kontrollproteins in den Proben A2, A3 usw. stehen über einen bestimmten Faktor mit dem aus Probe A1 im Verhältnis. Dieser Faktor kann verwendet werden, um die Signale des Zielproteins zu normalisieren. Eine mögliche Anwendung ist z.B. der quantitative Vergleich verschiedener Fällungsmethoden/-bedingungen für eine bestimmte Proteinprobe. Des Weiteren kann das Kontrollprotein zur Bestimmung der Gesamtproteinkonzentration dienen. Ist die Proteinkonzentration der ursprünglichen Proteinpräparation (bei Zugabe des Kontrollproteins) sowie die Konzentration des Kontrollprotein in der zu analysierenden Probe bekannt und ist diese ebenfalls im Gel aufgetrennt, kann dies verwendet werden, um die Konzentration nach Reinigung, Auftrennung usw. zu ermitteln. Beispielsweise kann das Bandenvolumen (bzw. Signalintensität) x des Kontrollproteins in der ursprünglichen Probe zu dem Bandenvolumen y des Kontrollproteins in der gereinigten Probe über einen bestimmten Faktor (x/y) miteinander im Verhältnis stehen. Demzufolge wird auch die Gesamtproteinkonzentration c₁ in der ursprünglichen Probe mit dem gleichen Faktor im Verhältnis zur Gesamtproteinkonzentration c₂ nach erfolgter Reinigung, Auftrennung usw. stehen. Eine zeitaufwändige Bestimmung der Proteinkonzentrationen einzelner Reinigungsstufen kann somit durch ein einzelnes Kontrollprotein ersetzt werden.

### Effizienz von Markierungsreaktionen (vorzugsweise mit Fluoreszenzfarbstoffen)

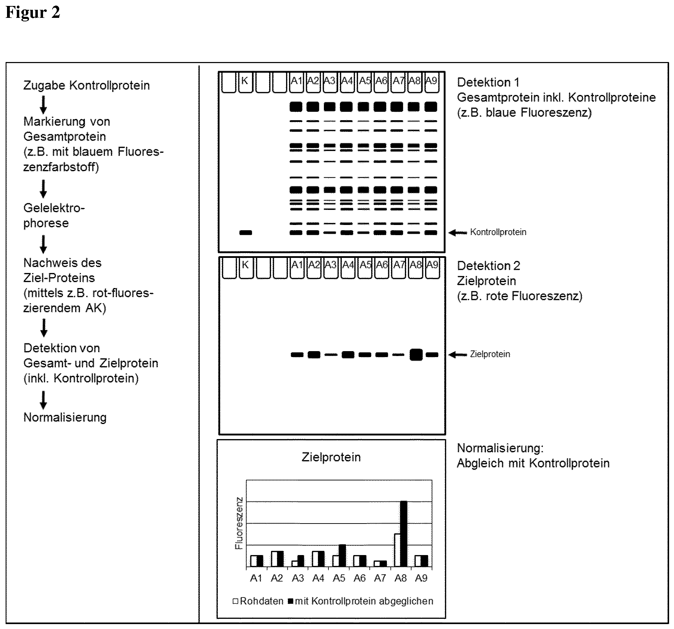

Markierungsreaktionen werden u.a. eingesetzt zur Visualisierung elektrophoretisch aufgetrennter Proteine. Dies wiederum dient der Quantifizierung einer oder mehrerer Proteinbanden innerhalb einer Probe oder - meist wichtiger - dem Vergleich mehrerer verschiedener Proteinproben (z.B. Quantifizierung eines Zielproteins in Proteinpräparationen von gesunden vs. kranken Patienten). Chemische Markierungsreaktionen können aber je nach Art der Probe und Probenpräparation unterschiedlich effizient ablaufen. Die Markierungsreaktion von Lysin-Seitenketten wird z.B. durch Anwesenheit von Aminen, die häufig Bestandteil von Puffersystemen sind, inhibiert. Durch Zugabe einer definierten Menge eines oder mehrerer Kontrollproteine zur Proteinprobe vor der Markierungsreaktion kann die Effizienz dieser Reaktionen verfolgt werden (siehe **Figur 2**). Da das extern zugegebene Kontrollprotein der gleichen Markierungsreaktion unterliegt wie die eigentliche Proteinprobe, können so die Signale des Kontrollproteins verwendet werden, um die Signale der Zielproteine zu normalisieren. Beispielsweise kann das Bandenvolumen x des Kontrollproteins in Probe A mit dem Bandenvolumen y des Kontrollproteins in Probe B über den Faktor x/y im Verhältnis stehen. Dieser Faktor wird verwendet, um die Bandenvolumina Proben-interner Proteine zu normalisieren und so unterschiedliche Markierungseffizienzen auszugleichen. Entscheidend für das Funktionieren eines Kontrollproteins ist, dass dieses visualisiert und kontrolliert werden kann. Es kann z.B. während der Probenvorbereitung durch simple Pipettierfehler zu einer ungleichmäßigen Beladung des ID-SDS-Gels kommen. Man würde dann unterschiedlich starke Kontrollproteinsignale erhalten, die jedoch nicht auf eine ungleichmäßige MarkierungsEffizienz zurückzuführen wären. Um dies auszuschließen, wird das Kontrollprotein - vor der eigentlichen Markierung- zusammen mit den Proteinproben - prä-markiert. Die Anregungs- und Emissionswellenlängen der verwendeten Fluoreszenzfarbstoffe müssen sich dabei ausreichend stark voneinander unterscheiden um eine getrennte Detektion zu ermöglichen.

### Quantifizierung von Targetproteinen

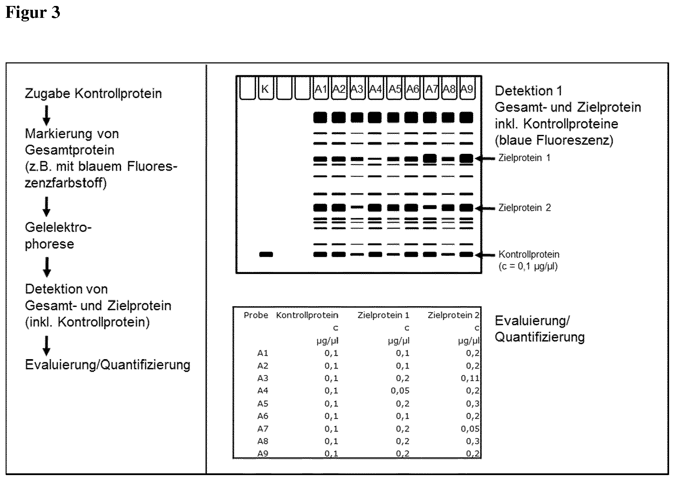

Der Einsatz eines Kontrollproteins zur Überwachung von Markierungsreaktionen kann gleichzeitig auch der Quantifizierung Proben-interner Zielproteine bzw. Zielprotein-Banden dienen (siehe **Figur 3**). Hierbei entspricht das Bandenvolumen (abzüglich Hintergrund) des Kontrollproteins der eingesetzten Konzentration dieses Proteins im Ansatz. Beispielsweise entspricht die Zugabe von 1 µg Kontrollprotein zu 100 µl zu analysierender Probe einer Kontrollprotein-Konzentration von 0,01 µg/µl. Somit entspricht das Bandenvolumen x des Kontrollproteins 0,01 µg/µl. Das Bandenvolumen y eines Zielproteins steht nun in einem bestimmten Verhältnis zum Bandenvolumen x. Im selben Verhältnis stehen auch die Konzentrationen von Kontroll- und Zielprotein, was die absolute Quantifizierung des Zielproteins erlaubt.

### Standardisierung von Laufverhalten/Trennstrecke

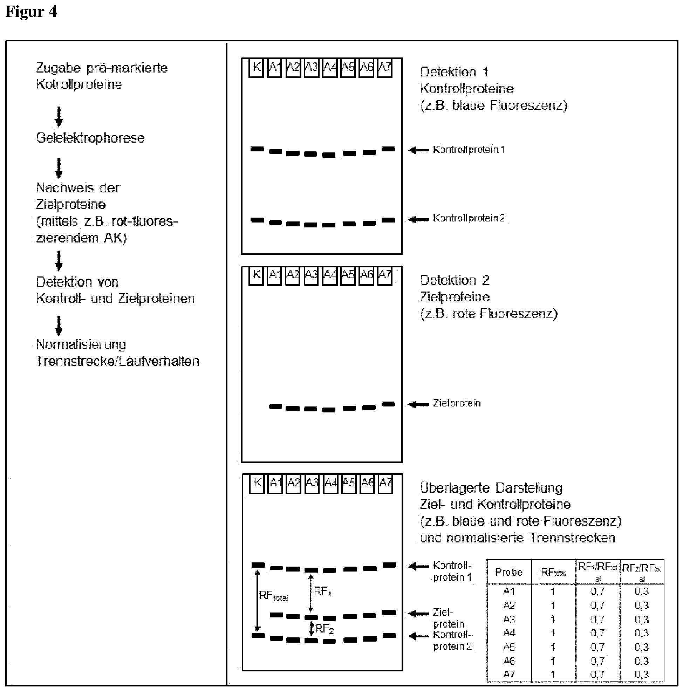

Während einer gel-elektrophoretischen Auftrennung von Protein-haltigen Proben kann es zu Ungleichmäßigkeiten des Laufverhaltens innerhalb eines Gels kommen (z.B. durch ungleichmäßige Kühlung, d.h. den sogenannten Smiling-Effekt). Des Weiteren unterscheiden sich die Laufverhalten verschiedener Gele voneinander. Dies führt zu unterschiedlichen Trennstrecken der jeweiligen Zielproteine in verschiedenen Proben. Dies erschwert die eindeutige Identifizierung von Zielproteinen, im Besonderen, wenn zwei Zielproteine mit ähnlichen Eigenschaften identifiziert und voneinander diskriminiert werden sollen. Der Einsatz prä-markierter Kontrollproteine ermöglicht die Kontrolle und Standardisierung des Laufverhaltens bzw. der Trennstrecke verschiedener Proben innerhalb eines Gels und/oder über mehrere Gele (siehe **Figur 4**). Unabhängig vom Gesamt-Laufverhalten des Gels wird der Abstand der beiden Kontrollproteine (RFₜₒₜₐₗ) gleich 1 gesetzt. Das Verhältnis des Abstandes beiden Kontrollproteine (RFₜₒₜₐₗ) zum Abstand zwischen oberem Kontrollprotein und Zielprotein (RF1) bzw. zum Abstand zwischen unterem Kontrollprotein und Zielprotein (RF2) sind unabhängig vom Laufverhalten immer gleich. So kann ein Zielprotein bei unterschiedlichen Laufweiten/Trennstrecken immer eindeutig identifiziert werden.

### Fluoreszenz-basierte inter-Assay Vergleiche (Gel-zu-Gel-Vergleiche)

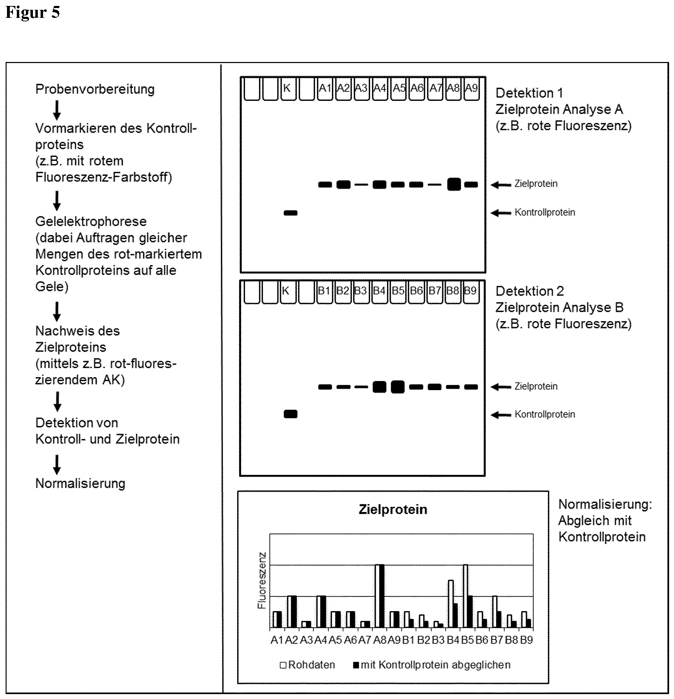

Der Einsatz von prä-markierten Kontrollproteinen erlaubt ebenfalls den Vergleich mehrerer Analysen (verschiedene Gele, verschiedene Blots, siehe **Figur 5**). Ist auf jedem dieser Gele bzw. Blots mindestens ein prä-markiertes Kontrollprotein in bekannter Menge aufgetragen, kann die entsprechende Bande bzw. deren Volumen genutzt werden, um mehrere Analysen/Gele/Blots miteinander in Beziehung zu setzen. Dabei muss das Kontrollprotein mit dem gleichen Fluoreszenz-Farbstoff prä-markiert sein wie die zu analysierenden Proben bzw. Zielproteine. Das Verhältnis des Bandenvolumens x des Kontrollproteins auf einem ersten Gel zum Bandenvolumen y desselben Kontrollproteins auf einem zweiten Gel wird beispielsweise verwendet, um die Fluoreszenz-Signale der zu analysierenden Proben bzw. Zielproteine auf den verschiedenen Gelen in Beziehung zu bringen (x/y).

### Kontrolle von Antikörper-Reaktionen bei Western-Blot Analysen

In der Regel erfolgt der Nachweis von Zielproteinen mittels Western-Blot Analysen unter Nutzung spezifischer Antikörper. In den meisten Fällen erfolgen während der gesamten Western Blot-Prozedur keine Zwischenkontrollen (z.B. nach Gel-Lauf oder Proteintransfer auf die Membran), so dass ein fehlendes Antikörper-Signal diverse Ursachen haben kann. Durch Einsatz prä-markierter Kontrollproteine kann eine ungleichmäßige Beladung visualisiert und ausgeglichen werden. Durch Fluoreszenz-Markierung von Gesamtprotein können Elektrophorese als auch Proteintransfer kontrolliert werden, wobei die vorherige Zugabe eines Kontrollproteins entscheidend ist, um unterschiedliche Markierungseffizienzen auszugleichen. Die Bindung und Reaktion des Antikörpers jedoch nicht. Hierfür eignen sich ebenfalls prämarkierte Kontrollproteine, die mit in die Analyse einbezogen werden. Ein Kontrollprotein sollte dabei den primären Antikörper binden und ein weiteres den sekundären Antikörper. Die Prä-Markierung (vorzugsweise mit einem Fluoreszenzfarbstoff, die sich von der Fluoreszenz des Antikörper-Konjugates unterscheidet) ermöglicht die Kontrolle der gleichmäßigen Beladung und Transfers sowie inter-Assay-Vergleiche. Durch den Einsatz prä-markierter Kontrollproteine kann eine mehrstufige Normalisierung innerhalb eines Gels/Blots erfolgen bis hin zur Gel/Blot-übergreifenden Normalisierung, was eine standardisierbare Auswertung - besonders für Analysen mit sehr großem Probenumfang - erst möglich macht.

### Ausführungsbeispiele

### Beispiel 1: Kontrolle einer Fluoreszenz-Markierungsreaktion (siehe Figur 6)

Markierungsreaktionen von Aminosäureseitenketten werden durch verschiedenste Faktoren sowohl positiv als auch negativ beeinflusst. Beta-Mercaptoethanol z.B. inhibiert die Labelingreaktion von Lysinseitenketten von Proteinen mit NHS-aktivierten Fluoreszenzfarbstoffen und diente im folgenden Versuch zur Simulation von unterschiedlich effizienter Markierung und deren Visualisierung durch ein Kontrollprotein. Als Kontrollprotein diente bovines Lactalbumin (Sigma-Aldrich). Zu 100 µl einer 1:20 (mit 50 mM Tris-HCl, pH 8,8) verdünnten Proteinprobe (humanes Blutserum) wurden 10 µg prä-markiertes Kontrollprotein zugegeben (c_{Kontrollprotein} = 0,1 µg/µl; c_{Serumprotein} = 5 µg/µl). 10 µl dieser Mischung (+/- 1% beta-Mercaptoethanol) wurden mit je 400 pmol (NHS-aktiviertem) T-Red (rote Fluoreszenz; NH DyeAGNOSTICS) markiert (30 min auf Eis), je 5 µl davon elektrophoretisch mittels 1D-SDS-PAGE aufgetrennt und die Fluoreszenz mit einem Fluoreszenz-Scanner (Fujifilm FLA-9000) detektiert. Im selben SDS-Gel erfolgte die Auftrennung von 0,5 µg mit T-Red gelabeltem Kontrollprotein (entspricht der Menge Kontrollprotein, die in den entsprechenden Serumproben enthalten waren). Die Ermittlung der Bandenvolumina erfolgte mit der Software LabImage 1D (Kapelan).

### Ergebnisse

Betrachtet man ausschließlich die Fluoreszenzsignale der Proben-internen Proteine, ergibt sich für die Serumprobe mit beta-Mercaptoethanol ein um das ca. 50fache schwächeres Fluoreszenzsignal im Vergleich zur Serumprobe ohne beta-Mercaptoethanol. Man würde also von einem deutlich geringen Proteingehalt der Probe mit Mercaptoethanol ausgehen, obwohl für beide Proben die gleiche Menge markiert und auf dem SDS-Gel aufgetrennt wurde. Erst der Zusatz des Kontrollproteins ermöglicht eine Visualisierung und Normalisierung der unterschiedlichen Markierungseffizienz.

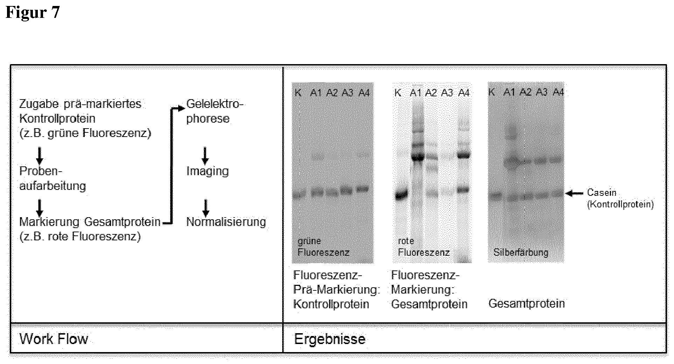

### Beispiel 2: Kontrolle des Kontrollproteins (Figur 7)

Entscheidend für das Funktionieren eines Kontrollproteins ist, dass dieses visualisiert und kontrolliert werden kann. Versuch 2 demonstriert die Kontrolle des Kontrollproteins. Als Kontrollprotein diente bovines beta-Casein (Sigma-Aldrich). 50 µg Kontrollprotein (in 50 mM Tris-HCl, pH 8,8) wurden mit 400 pmol (NHS-aktiviertem) T-Green (grüne Fluoreszenz; NH DyeAGNOSTICS) markiert. Je 10 µg prä-markiertes Kontrollprotein wurden zu 100 µl humaner Proteinprobe (A1 bis A4) zugegeben (c_{Kontrollprotein} = 0,1 µg/µl; Cₕᵤₘₐₙₚᵣₒₜₑᵢₙ = 0,5 bis 2 µg/µl). Je 10 µl Probe A1 bis A4 sowie 50 µg pre-markiertes Kontrollprotein wurden mit 400 pmol (NHS-aktiviertem) T-Red (rote Fluoreszenz) markiert. Es wurden je 5 µl Probe (A1 bis A4) und 0,5 µg doppelt-markiertes Kontrollprotein (entspricht der Menge Kontrollprotein, die in den entsprechenden Humanproben enthalten waren) elektrophoretisch mittels 1D-SDS-PAGE aufgetrennt. Die Fluoreszenz wurde mittels Fluoreszenz-Scanner (Fuji-Film FLA-9000) detektiert und die Bandendetektion und Quantifizierung erfolgte mit LabImage 1D (Kapelan). Zur Kontrolle erfolgte eine Silberfärbung der aufgetrennten Proteine.

### Ergebnis

Die Detektion im grünen Fluoreszenzkanal zeigt die Prä-Markierung des Kontrollproteins mit T-Green. Dessen gleichmäßiges Vorhandensein spricht für einen gleichmäßigen Probenauftrag. Die Detektion im roten Fluoreszenz-Signal zeigt den Marker des Gesamt- und des Kontrollproteins. Die ungleichmäßige Bandenintensität spricht für eine unterschiedliche Markierungseffizienz der einzelnen Proteinproben. Nachträgliche Silberfärbung bestätigt das homogene Vorhandensein des doppelt-markierten Kontrollproteins.

### Referenzbeispiel 3: Kontrollproteine für den Gel-zu-Gel-Vergleich (Figur 8)

Fluoreszierende Proteinproben auf unterschiedlichen 1D-SDS-Gelen miteinander zu vergleichen, ist nicht ohne weiteres möglich. Zu möglichen unterschiedlichem Laufverhalten kommen Unterschiede in den Detektionsparametern. Um für eine optimale Quantifizierung möglichst alle zur Verfügung stehenden Graustufen (bei 16bit Tiff: ca. 65000), erfolgt das Fluoreszenz-Imaging so, dass das stärkste Signal kurz unterhalb der Sättigung ist. Je nach Proben und Probentyp kann dieses von Gel zu Gel variieren. Der Einsatz einer definierten Menge an prä-markiertem Kontrollprotein auf jedem Gel ermöglicht die beiden Gele miteinander in Beziehung zu setzen.

Im folgenden Versuch dienten bovines Serumalbumin (BSA; Serva) und bovines beta-Casein (Sigma-Aldrich) als Kontrollproteine und ein Gemisch aus 4 Standard-Proteinen - darunter bovines beta-Casein - als simulierte Proteinprobe S1. Je 50 µg Kontrollproteingemisch bzw. Standardprotein-Gemisch (in 50 mM Tris-HCl, pH 8,8) wurden mit je 400 pmol (NHS-aktiviertem) T-Red (rote Fluoreszenz) markiert (30 min auf Eis). Je 0,5 µg Kontrollprotein und 5 µl Standardproteingemisch wurden elektrophoretisch auf 2 verschiedenen 1D-SDS-Gelen aufgetrennt. Die Fluoreszenz wurde mittels NHD Octoplus QPLEX Fluoreszenz-Imager mit den für das jeweilige Gel optimalen Belichtungszeiten (stärkstes Signal kurz unterhalb der Sättigung) detektiert. Die Bestimmung der Bandenvolumina erfolgte mit der Software Lab Image 1D (Kapelan). Die Beziehung zwischen beiden Gelen wurde über die beiden Kontrollproteine hergestellt. Bandenvolumen x des Kontrollproteins in Gel A steht mit einem bestimmten Faktor im Verhältnis zum Bandenvolumen y des Kontrollproteins in Gel B (Faktor = x/y). Mit diesem Faktor kann auch das Bandenvolumen v von beta-Casein in S1 in Gel A zu dem Bandenvolumen z von beta-casein in S 1 in Gel B normalisiert werden.

### Ergebnis

Geht man nur von den gemessenen Bandenrohvolumina für beta-Casein in S1 aus, würde die Bande in Gel B deutlich stärker ausfallen als in Gel A (obwohl gleiche Proteinmengen aus ein und derselben Probe aufgetragen wurden). Erst nach Abgleich mit den Kontrollproteinen erhält man gleiche Bandenvolumina für beta-Casein in S1 in beiden Gelen.

## Patentansprüche

1. Verfahren zum Überwachen und zur Kontrolle der Probenvorbereitung, Probenreinigung und/oder Probenauftrennung biologischer Proben sowie von chemischen Markierungsreaktionen und Antikörperreaktionen in einer Probe, die Proteine enthält, umfassend die Schritte:
i. Zugabe eines Kontrollproteins zu der Probe, wobei das Kontrollprotein vor Zugabe zu der Probe markiert wurde,
ii. optional Schritte der Probenvorbereitung , z. B. Proteinfällungen,
iii. Markierung des wenigsten einen Zielproteins und des markierten Kontrollproteins, vorzugsweise mit einem Fluoreszenz-Farbstoff, in der Probe,
iv. Auftrennung der Proteine in der Probe mittels Gelelektrophorese zur Separation des wenigstens einen markierten Zielproteins und des doppelt-markierten Kontrollproteins,
v. Nachweis und/oder Quantifizieren des wenigstens eines Zielproteins und des markierten Kontrollproteins,
vi. Normalisierung der quantifizierten Menge und/oder Konzentration und/oder des Laufverhaltens/der Trennstrecke und/oder absolute Quantifizierung des wenigstens einen Zielproteins anhand der bekannten Menge und/oder Konzentration und/oder des Laufverhaltens/der Trennstrecke des markierten Kontrollproteins in der Probe,
**dadurch gekennzeichnet, dass** das Kontrollprotein bei der Markierung des Zielproteins doppelt markiert wird und das Kontrollprotein mit verschiedenen Farbstoffen markiert wird.

2. Verfahren nach Anspruch 1, wobei das Kontrollprotein das gleiche Protein wie das Zielprotein ist oder wobei das Kontrollprotein von dem Zielprotein verschieden ist.

3. Verfahren nach einem der Ansprüche 1 bis 2, wobei die Fluoreszenzfarbstoffe kovalent an das Zielprotein und das Kontrollprotein gebunden sind.

4. Verfahren nach einem der Ansprüche 1 bis 3, wobei das Kontrollprotein ein natürliches Protein, ein rekombinantes Protein oder ein synthetisch hergestelltes Protein ist.

5. Verfahren nach einem der Ansprüche 1 bis 4, wobei die Probe ausgewählt sein kann aus Vollblut, Blutserum oder Plasma, Urin, Speichel, Hirnflüssigkeit (CSF), anderen Körperflüssigkeiten , wie z. B. Stuhl, Tränenflüssigkeit, Gelenkflüssigkeit, Sputum, Atem, z .B. als kondensierter Atem, oder einem Extrakt oder einer Aufreinigung oder einer Verdünnung davon, Gewebehomogenate, Gewebesektionen und Biopsien.

6. Verfahren nach einem der Ansprüche 1 bis 5, wobei nach der Auftrennung der Proteine mittels Gelelektrophorese ein Western-Blot durchgeführt wird.

7. Verfahren nach einem der Ansprüche 1 bis 6, wobei die Normalisierung zwischen wenigstens einem Zielprotein und wenigstens einem Kontrollproteinen erfolgt, die auf dem gleichen Elektrophoresegel und/oder der gleichen Blotting-Membran aufgetragen worden sind (intra-Assay)
oder
wobei die Normalisierung zwischen wenigstens einem Zielprotein und/oder wenigstens einem Kontrollproteinen erfolgt, die auf verschiedenen Elektrophoresegelen und/oder verschiedenen Blotting-Membranen aufgetragen worden sind (inter-Assay).

8. Verwendung des Verfahrens nach einem der Ansprüche 1 bis 7 zum Überwachen der Probenvorbereitung, Probenreinigung und/oder Probenauftrennung proteinhaltiger Proben.

9. Verwendung nach Anspruch 8 zur
• quantitativen Kontrolle und/oder Visualisierung von Proteinverlusten während der Probenvorbereitung und/oder Proteinreinigung und/oder Gelbeladung,
• quantitativen Kontrolle der Effizienz chemischer Markierungsreaktionen, z .B. mit Fluoreszenz -Farbstoffen,
• absoluten Quantifizierung des Gehaltes eines oder mehrerer Zielproteine innerhalb einer Probe oder zwischen verschiedenen Assays (intra-Assay),
• Durchführung von Gel-zu-Gel-Vergleichen und/oder -Normalisierungen,
• Kontrolle und Standardisierung des Laufverhaltens/der Trennstrecke verschiedener Proben innerhalb eines Gels sowie auch Gel-übergreifend,
• quantitativen Kontrolle von Antikörper-Reaktionen bei Western-Blott Analysen, und/oder
• zum Ausschluss falsch-positiver bzw. falsch-negativer Messergebnisse.

## Claims

1. Method for monitoring and controlling sample preparation, sample purification and/or sample fractionation of biological samples and chemical labelling reactions and antibody reactions in a sample containing proteins, comprising the following steps:
i. adding a control protein to the sample, which control protein has been labelled prior to addition to the sample,
ii. optionally steps of sample preparation, for example protein precipitations,
iii. labelling the at least one target protein and the labelled control protein, preferably with a fluorescent dye, in the sample,
iv. fractionating the proteins in the sample by means of gel electrophoresis to separate the at least one labelled target protein and the twice labelled control protein,
v. detecting and/or quantifying the at least one target protein and the labelled control protein,
vi. normalizing the quantified amount and/or concentration and/or the migration behaviour/separating path of and/or quantifying absolutely the at least one target protein based on the known amount and/or concentration and/or the migration behaviour/separating path of the labelled control protein in the sample,
**characterized in that** the control protein is labelled twice during labelling of the target protein and the control protein is labelled with different dyes.

2. Method according to Claim 1, wherein the control protein and the target protein are the same or wherein the control protein and the target protein are different.

3. Method according to either of Claims 1 and 2, wherein the fluorescent dyes are covalently attached to the target protein and the control protein.

4. Method according to any of Claims 1 to 3, wherein the control protein is a natural protein, a recombinant protein or a synthetically produced protein.

5. Method according to any of Claims 1 to 4, wherein the sample may be selected from whole blood, blood serum or plasma, urine, saliva, cerebrospinal fluid (CSF), other body fluids such as, for example, faeces, lacrimal fluid, synovial fluid, sputum, breath, for example as breath condensate, or an extract or a purification or a dilution therefrom, tissue homogenates, tissue sections, and biopsies.

6. Method according to any of Claims 1 to 5, wherein Western blotting is carried out following fractionation of the proteins by means of gel electrophoresis.

7. Method according to any of Claims 1 to 6, wherein normalization is performed between at least one target protein and at least one control protein, which have been applied to the same electrophoretic gel and/or the same blotting membrane (intra-assay), or
wherein normalization is performed between at least one target protein and/or at least one control protein, which have been applied to different electrophoretic gels and/or different blotting membranes (inter-assay).

8. Use of the method according to any of Claims 1 to 7 for monitoring sample preparation, sample purification and/or sample fractionation of proteinaceous samples.

9. Use according to Claim 8 for
• quantitatively controlling and/or visualizing protein losses during sample preparation and/or protein purification and/or gel loading,
• quantitatively controlling the efficiency of chemical labelling reactions, for example with fluorescent dyes,
• quantifying absolutely the content of one or more target proteins within a sample or between different assays (intra-assay),
• performing gel-to-gel comparisons and/or normalizations,
• controlling and standardizing the migration behaviour/separating path of different samples within a single gel and also between gels,
• quantitatively controlling antibody reactions in Western-blot analyses, and/or
• to exclude false-positive and false-negative measurement results.

## Revendications

1. Procédé de surveillance et de contrôle de la préparation, de la purification et/ou de la séparation d'échantillons biologiques, ainsi que de réactions de marquage chimiques et de réactions d'anticorps dans un échantillon qui contient des protéines, comprenant les étapes suivantes :
i. l'ajout d'une protéine de contrôle à l'échantillon, la protéine de contrôle ayant été marquée avant l'ajout à l'échantillon,
ii. éventuellement des étapes de préparation de l'échantillon, p. ex. des précipitations de protéines,
iii. le marquage de ladite au moins une protéine cible et de la protéine de contrôle marquée, de préférence avec un colorant de fluorescence, dans l'échantillon,
iv. la séparation des protéines dans l'échantillon par électrophorèse sur gel pour la séparation de ladite au moins une protéine cible marquée et de la protéine de contrôle doublement marquée,
v. la détection et/ou la quantification de ladite au moins une protéine cible et de la protéine de contrôle marquée,
vi. la normalisation de la quantité et/ou concentration quantifiée et/ou de la migration/distance de séparation et/ou quantification absolue de ladite au moins une protéine cible à partir de la quantité et/ou concentration connue et/ou de la migration/distance de séparation de la protéine de contrôle marquée dans l'échantillon,
**caractérisé en ce que** la protéine de contrôle est doublement marquée lors du marquage de la protéine cible et la protéine de contrôle est marquée avec différents colorants.

2. Procédé selon la revendication 1, dans lequel la protéine de contrôle est la même protéine que la protéine cible ou dans lequel la protéine de contrôle est différente de la protéine cible.

3. Procédé selon l'une quelconque des revendications 1 à 2, dans lequel les colorants de fluorescence sont reliés de manière covalente à la protéine cible et à la protéine de contrôle.

4. Procédé selon l'une quelconque des revendications 1 à 3, dans lequel la protéine de contrôle est une protéine naturelle, une protéine recombinante ou une protéine fabriquée par synthèse.

5. Procédé selon l'une quelconque des revendications 1 à 4, dans lequel l'échantillon peut être choisi parmi le sang total, le sérum sanguin ou le plasma, l'urine, la salive, le liquide cérébro-spinal (LCS), d'autres fluides corporels, tels que p. ex. les selles, le liquide lacrymal, le liquide synovial, les expectorations, la respiration, p. ex. sous la forme de respiration condensée, ou un extrait ou une purification ou une dilution de celle-ci, les homogénats de tissus, les sections de tissus et les biopsies.

6. Procédé selon l'une quelconque des revendications 1 à 5, dans lequel un buvardage de western est réalisé après la séparation des protéines par électrophorèse sur gel.

7. Procédé selon l'une quelconque des revendications 1 à 6, dans lequel la normalisation a lieu entre au moins une protéine cible et au moins une protéine de contrôle, qui ont été appliquées sur le même gel d'électrophorèse et/ou la même membrane de buvardage (intra-essai),
ou
dans lequel la normalisation a lieu entre au moins une protéine cible et/ou au moins une protéine de contrôle, qui ont été appliquées sur différents gels d'électrophorèse et/ou différentes membranes de buvardage (inter-essai).

8. Utilisation du procédé selon l'une quelconque des revendications 1 à 7 pour la surveillance de la préparation, de la purification et/ou de la séparation d'échantillons contenant des protéines.

9. Utilisation selon la revendication 8 pour
- le contrôle quantitatif et/ou la visualisation de pertes de protéines pendant la préparation d'échantillons et/ou la purification de protéines et/ou le chargement d'un gel,
- le contrôle quantitatif de l'efficacité de réactions de marquage chimiques, p. ex. avec des colorants de fluorescence,
- la quantification absolue de la teneur en une ou plusieurs protéines cibles dans un échantillon ou entre différents essais (intra-essai),
- la réalisation de comparaisons et/ou de normalisations gel à gel,
- le contrôle et la normalisation de la migration/distance de séparation de différents échantillons dans un gel et également entre plusieurs gels,
- le contrôle quantitatif de réactions d'anticorps lors d'analyses de buvardage de western, et/ou
- pour l'exclusion de résultats de mesure faussement positifs ou faussement négatifs.
